# EUROPÄISCHE PATENTSCHRIFT

(11) **EP 2 091 930 B1**
(45) Veröffentlichungstag und Bekanntmachung des Hinweises auf die Patenterteilung: **30.05.2012**
(21) Anmeldenummer: 07822127.2
(22) Anmeldetag: 31.10.2007
(51) Int. Cl.: C07D 295/02, C07D 295/027, C08K 5/378, C08K 5/43, C08K 5/00

(54) **VERNETZER ZUR VULKANISATION, VERFAHREN ZU DESSEN HERSTELLUNG SOWIE GUMMIERZEUGNISSE**
CROSSLINKER FOR VULCANIZATION, PROCESS FOR ITS PREPARATION AND RUBBER PRODUCTS
AGENT RÉTICULANT POUR VULCANISATION, SON PROCÉDÉ DE FABRICATION ET PRODUITS EN CAOUTCHOUC

(30) Priorität: 31.10.2006 DE 102006052197
(43) Veröffentlichungstag der Anmeldung: 26.08.2009
(73) Patentinhaber: Gribov, Iourii, 10829 Berlin (DE); Kazimirskiy, Pavel, 13591 Berlin (DE)
(72) Erfinder: Gribov, Iourii, 10829 Berlin (DE); Kazimirskiy, Pavel, 13591 Berlin (DE)
(74) Vertreter: Kietzmann, Manfred
(86) Internationale Anmeldenummer: PCT/EP2007/061780
(87) Internationale Veröffentlichungsnummer: WO 2008/053023

(56) Entgegenhaltungen:
- EP-A- 0 481 366
- US-A- 4 632 988
- ROSTEK C J ET AL: "NOVEL SULFUR VULCANIZATION ACCELERATORS BASED ON MERCAPTO-PYRIDINE,-PYRAZINE, AND -PYRIMIDINE" RUBBER CHEMISTRY AND TECHNOLOGY, ACS, AKRON, OH, US, Bd. 69, Nr. 2, 1. Mai 1996 (1996-05-01), Seiten 180-202, XP000597608 ISSN: 0035-9475
- DATTA ET AL: "Studies on the reactions between thiocarbamyl sulfenamide and 2-(iminodithio)benzothiazole accelerator system in the early stage of vulcanization of NR" J. APPL. POLYMER SCI., Bd. 32, 1986, Seiten 5849-5864, XP002470142
- SHARADA ET AL: ZEITSCHRIFT FUR ANORGANISCHE UND ALLGEMEINE CHEMIE, vol. 304, 1960, pages 344-350,

## Beschreibung

Die Erfindung betrifft einen Vernetzer zur Vulkanisation, ein Verfahren zu dessen Herstellung sowie ein entsprechend hergestelltes Gummierzeugnis.

Neben der Vulkanisation mit elementarem Schwefel, die als Heißvulkanisation bezeichnet wird, existiert die Möglichkeit bei niedriger Temperatur zu vernetzen. Dies wird als Niedertemperatur-Vulkanisation bezeichnet. Hierbei wird üblicherweise satt Schwefel in Schwefelkohlenstoff gelöstes Schwefeldichlorid oder Dischwefeldichlorid eingesetzt.

Die Niedertemperatur-Vulkanisation (T<100° C) wurde früher vor allem zur Herstellung dünner Schichten verwendet. Gummihandschuhe wurden z.B. durch Eintauchen eines Formkörpers in eine Latexemulsion und anschließendes Eintauchen in eine Lösung von S₂Cl₂ in CS₂, Benzin oder Benzol hergestellt. Dabei entstehen Brücken, die nur ein Schwefelatom enthalten. In einer NH₃-Atmosphäre wird die entstandene Salzsäure neutralisiert und das überschüssige Dischwefeldichlorid zersetzt.

Zur Beschleunigung der Vulkanisation von Gummimischungen auf der Basis von Kautschuk sind eine Reihe von Beschleuniger bekannt, z.B. Mercaptobenzothiazol, Sulfenamid, Tetramethyltiuram.

In der Veröffentlichung von Sharada et al "Piperidine Thiosulfphate" (Zeitschrift für anorganische und allgemeine Chemie, Bd. 304, 1960, Seiten 344 - 350) ist die Herstellung von Piperidinthiosulfat beschrieben. Hierzu wird 1 -2% Schwefel in Piperidin gelöst, wobei sich rote lösliche Piperidinsulfide bilden, die durch molekularen Sauerstoff oxidiert werden. Dabei kristallisiert Piperidinthiosulfat mit geringen Beimengungen von Piperidinsulfit und Piperidinsulfat aus. Die Reaktion wird mit niedrigen Schwefelkonzentrationen und ohne Kontrolle der Reaktionstemperatur ausgeführt.

US-A-4 632 988 offenbart ein Verfahren zur Herstellung von Piperidinsulfiden ausgehend von Piperidin und Schwefel in Methanol am Rückfluss gefolgt von der Zugabe von NaOCl.

EP-A-0 481 366 offenbart ein Verfahren zur Herstellung von Piperidinodithiobenzothiazol ausgehend von Piperidin und Schwefel in Methanol in Gegenwart von Cu(OAc)₂, Ammoniaklösung und Sauerstoff bei 30°C.

RUBBER CHEMISTRY AND TECHNOLOGY, Bd. 69, Nr. 2, 1996, Seiten 180-202 und J. APPL. POLYMER SCI., Bd. 32, 1986, Seiten 5849-5864 offenbaren verschiedene Schwefelverbindungen und Gummierzeugnisse.

Es ist daher Aufgabe der Erfindung, einen Vernetzer für die Niedertemperatur - Vulkanisation bzw. ein Verfahren zur Herstellung eines derartigen Vernetzers zu schaffen, der bzw. das die Nachteile des Standes der Technik vermeidet.

Die Aufgabe wird durch einen Vernetzer, ein Verfahren zu dessen Herstellung sowie durch Gummierzeugnisse, die unter Verwendung des Vernetzers hergestellt wurden, und einen Vulhanisations prozeß gemäß Ansprüchen 1, 8, 12 und 14 gelöst.

Der erfindungsgemäße Vernetzer basiert auf einer Mischung aus stickstoffshaltigen, heterozyklischen Verbindungen und Schwefel, vorzugsweise in Pulverform.

Bei den heterozyklischen Verbindungen handelt es sich vorzugsweise um Piperidin, Pyridin, Anabassin und α α', β,'β, γ, γ'-Dipyridile und besonders bevorzugt um Piperidin.

Nach einer Ausführungsform der Erfindung kann neben den genannten heterozyklischen Verbindungen ein weiterer handelsüblicher Vernetzer oder Beschleuniger mit Schwefel umgesetzt werden.

Diese können beispielsweise aus der Gruppe Mercaptobenzothiazol, Sulfenamid, Tetramethyltiuram oder deren Derivate ausgewählt sein.

Die stickstoffshaltige, heterozyklische Verbindung wird mit Schwefel bei einer Temperatur unterhalb von 100°C umgesetzt, wodurch der gewünschte Vernetzer erhalten wird. Vorzugsweise wird die Reaktionstemperatur der Mischung unterhalb der Siedetemperatur der heterozyklischen Verbindung gehalten.

Für die Reaktion kann entweder die heterozyklische Verbindung oder der Schwefel vorgelegt werden.

Um die unter möglichst milden Bedingungen durchzuführen kann unter vermindertem Druck und/oder unter Kühlung gearbeitet werden.

Nach vollständiger Vermischung der beiden Komponenten wird die Temperatur, vorzugsweise unter Rühren noch weiter gehalten. Vorzugsweise bei 65° bis 70° C über einen Zeitraum von 2 bis 3 Stunden.

Das Vermischen erfolgt durch übliche Rührer oder Mischer, wie z. B. Zentrifugalmischer, Propellermischer, Magnetmischer, Ultraschallmischer und/oder Vibrationsmischer verwendet wird. Auch durch turbulente Strömungen kann eine Mischung erfolgen.

Die Mischung kann unter Schutzgas durch geführt werden.

Gegenstand dieser Erfindung sind der Vernetzer, das Verfahren zu seiner Herstellung sowie Gummierzeugnisse, die mittels des Vernetzers hergestellt wurden.

Gummierzeugnisse können erfindungsgemäß auch durch Verwendung des erfindungsgemäßen Vernetzers in Verbindung mit anderen handelsüblichen Vernetzern und/oder Beschleunigern hergestellt werden.

Die erfindungsgemäßen Verbindungen wirken neben der Reaktion des Schwefels zur Vulkanisierung als polyfunktionelle Agenzien. So können diese eine Rolle als Plastifikatoren und Stabilisatoren, zur Fixierung und/oder zur Modulation der Vulkanisierung, d. h. zur Sicherstellung eines definierten Vulkanisationsgrades und zur Beschleunigung des Vulkanisierungsprozesses, innehaben. Diese Aminstruktur dient zur Initiierung der Reaktion von freien Radikalen des Schwefels.

Der erfindungsgemäße Vernetzer kann sowohl zur Vulkanisation bei hohen Temperaturen als auch zur Tieftemperatur-Vulkanisation verwendet werden, wobei die Tieftemperatur-Vulkanisation besonders bevorzugt ist. Zur Vulkanisation können beliebige Gummimischungen eingesetzt werden, wie beispielsweise Gummimischungen auf Basis von natürlichem Kautschuk und synthetische Mischungen wie Chloropren-, Nitril-, Na-Butadien- ,Styrolkautschuke und dergleichen.

Die erfindungsgemäße Methode ist ökologisch vollkommen unbedenklich, wobei das gesamte Ausgangsmaterial zum Vernetzer umgesetzt wird. Die Umsetzung verläuft sehr schnell und wirtschaftlich vorteilhaft, wobei keine toxischen Hilfsstoffe verwendet werden müssen.

Der Vulkanisationsprozess mit dem erfindungsgemäßen Vernetzer weist gegenüber dem Stand der Technik verschiedene Vorteile auf:
1) Der Vernetzer arbeitet wie ein vulkanisierendes Mittel (Vulkanisationsagens) und ist fähig, die Gummimischung in der Abwesenheit von zusätzlichem, üblicherweise sehr unregelmäßig verteiltem Schwefelpulver zu vulkanisieren, was die Homogenität des der Vernetzung des Gummis sowie die Wärmebeständigkeit der Gummierzeugnisse erhöht.
2) Der erfindungsgemäße Vernetzer dient ebenfalls als Beschleuniger, da der Vulkanisationsprozess viel schneller verläuft. Die erhaltenen Gummimischungen erreichen verbesserte physikalisch-mechanische Messwerte.
3) Der Vernetzer weist die Eigenschaften von primären Plastifikatoren auf, was die Plastizität der Gummimischung vergrößert.
4) Der Vulkanisationsprozess benötigt kein Zusatz von Zinkoxid lässt und wird durch Abwesenheit von Zinkoxid nicht verzögert.
5) Die Zeit zur Mischung des Vernetzers mit der Rohgummimischung reduziert sich erheblich.
6) Der Vernetzer verteilt sich auch bei niedrigen Temperaturen gleichmäßiger in der Masse der Gummimischung in kurzer Zeit.
7) Der Vernetzer vulkanisiert die Gummimischungen bei der Temperatur niedriger 100° C. Er ist jedoch auch für die üblicherweise verwendeten Temperaturbereiche geeignet, wobei dann natürlich die Reaktionsgeschwindigkeit entsprechend erhöht ist.
8) Der Vernetzer vergrößert das Plateau der Vulkanisierung von üblicherweise Minuten bis hin zu Stunden.
9) Der Vernetzer verbessert die Ozon-Beständigkeit der Gummierzeugnisse.
10) Im Vergleich zum Stand der Technik können durch den erfindungsgemäßen Vernetzer bei der Vulkanisation beliebige Schichtdicken realisiert werden.

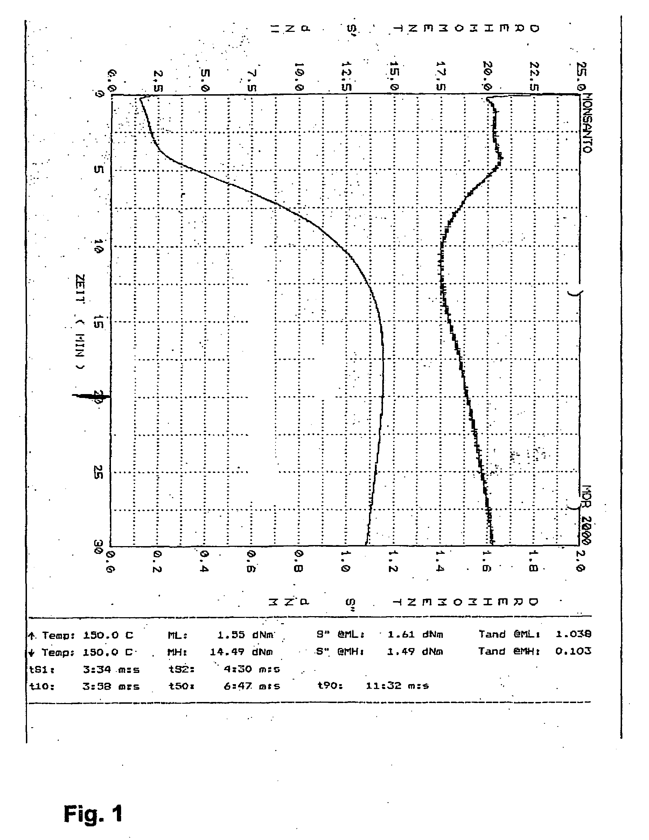

Nachstehend wird die Erfindung anhand von Beispielen und Figuren näher erläutert. Es zeigen
- Fig. 1: Vulkameterkurve einer Rohgummimischung nach dem Stand der Technik mit Schwefelpulver vernetzt (Mischung 1),

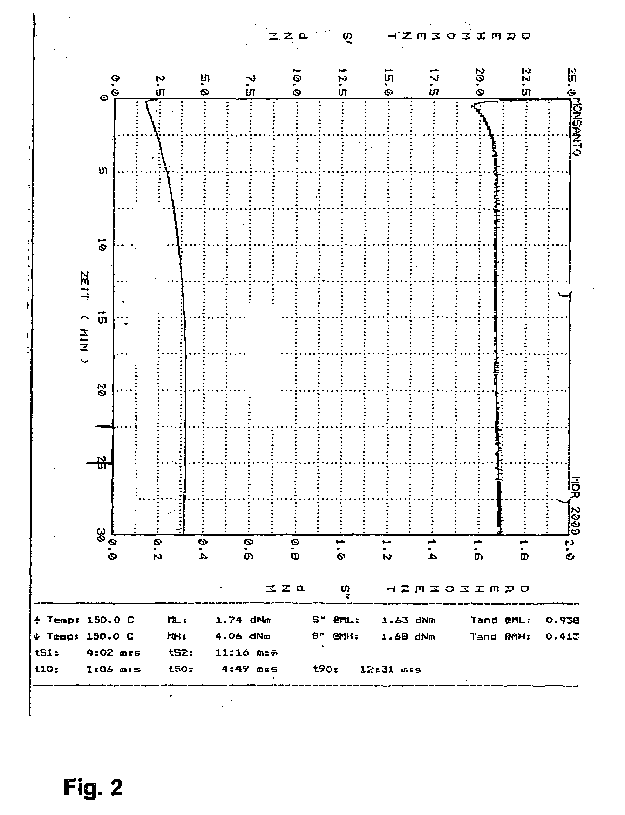

- Fig. 2: Vulkameterkurve der gleichen Rohgummimischung mit erfindungsgemäßen Vernetzer vernetzt (Mischung 2),

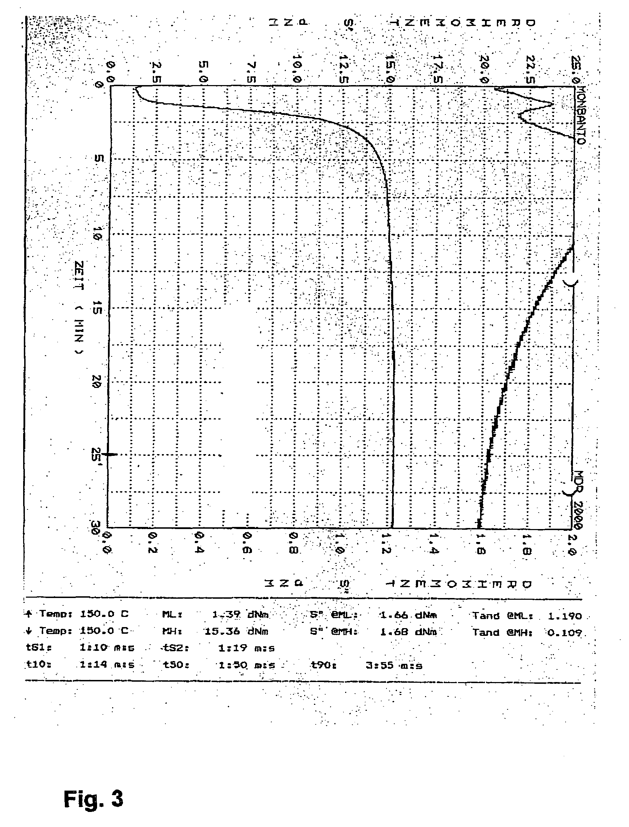

- Fig. 3: Vulkameterkurve der gleichen Rohgummimischung mit einem anderen Anteil an erfindungsgemäßen Vernetzer (Mischung 3),

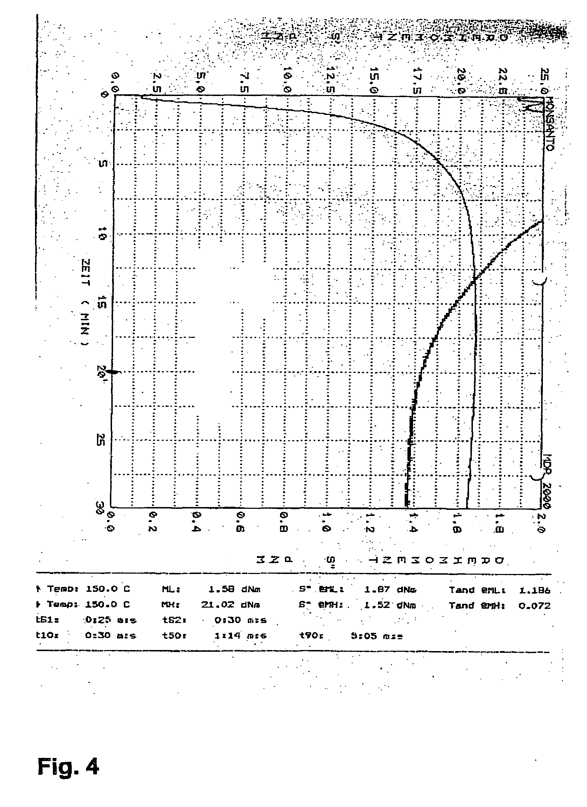

- Fig. 4: Vulkameterkurve der gleichen Rohgummimischung mit einem anderen Anteil an erfindungsgemäßen Vernetzer (Mischung 4),
- Fig. 5: ein IR Spektrum der gleichen Rohgummimischung nach dem Stand der Technik mit Schwefelpulver vernetzt (Mischung 1),
- Fig. 6: ein IR Spektrum eines erfindungsgemäßen Vernetzers,
- Fig. 7: ein IR Spektrum der gleichen Rohgummimischung mit erfindungsgemäßen Vernetzer vernetzt (Mischung 3),
- Fig. 8: Vergleich der Härte Shore A,
- Fig. 9: Vergleich der Reißfestigkeit und
- Fig. 10: Vergleich der Reißdehnung.

### Beispiel 1

### Synthese eines erfindungsgemäßen Vernetzers

Es werden 384 g Schwefel in Pulverform in einem Kolben gegeben, der mit einem Rührer sowie einem Thermometer versehen ist. Der Kolben wird in einem Kühlbad zur Wasserkühlung platziert. Unter Rühren werden 396 ml Piperidin zugetropft. Alternativ kann auf das Piperidin vorgelegt werden und das Schwefelpulver wird portionsweise zugemischt. Die Kühlung der Reaktionsmischung ist notwendig, da Reaktion exotermisch ist. Nach vollständiger Zugabe des Piperidins wird die Temperatur der Reaktionsmischung bei nicht mehr als 65° bis 70° C gehalten. Nach 2,5 h wird die Reaktionsmischung in einen Kunststoffbehälter umgegossen und auf 5° bis 10° C gekühlt. Das gekühlte Reaktionsgemisch weist die Konsistenz von kristallisiertem Honig auf und zeigt eine graubraun-rote Farbe. Der Schmelzbereich des Reaktionsprodukts liegt bei ca. 60° C.

Die Synthese kann auch als kontinuierliches Verfahren mittels turbulenter Durchmischung der zu mischenden Stoffe durchgeführt werden, wobei dies auch unter Schutzgas erfolgen kann.

### Beispiel 2

### IR-Spektroskopische Untersuchungen

### Gerät: Thermoelektron IR - Spektrometer Typ 380 mit ART - Einheit

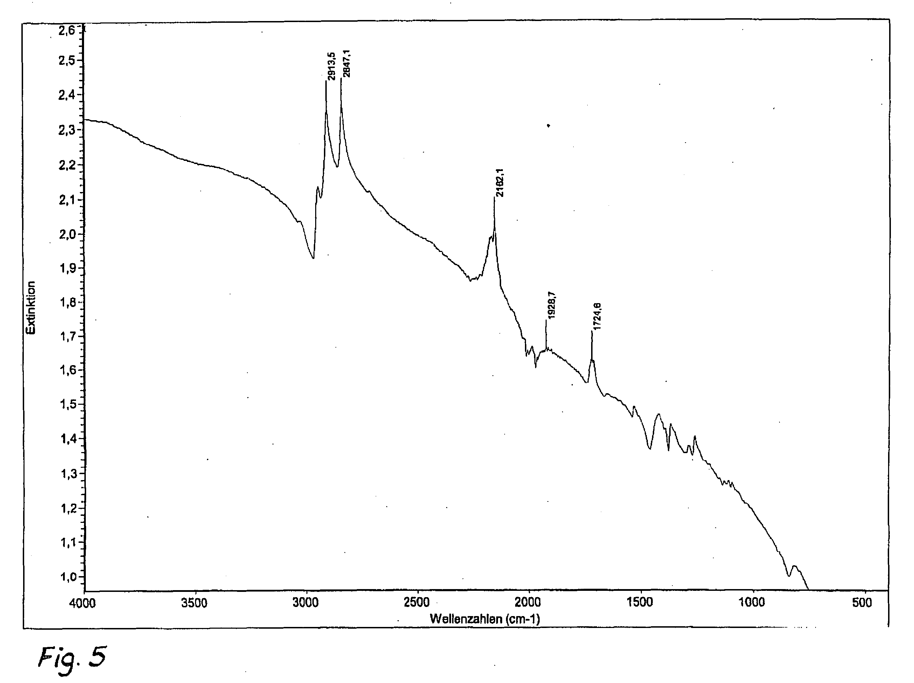

Das IR-Spektrum gemäß Fig. 5 zeigt die relativ schwache Merkmalausprägung der charakteristischen IR-Spektrallinien eine relativ strukturlose Vernetzung des Gummis nach dem Stand der Technik (Mischung 1). Die Ursache dafür besteht in der stets schlechten Vermischung des Schwefelpulvers in die sehr viskose Rohrgummimischung.

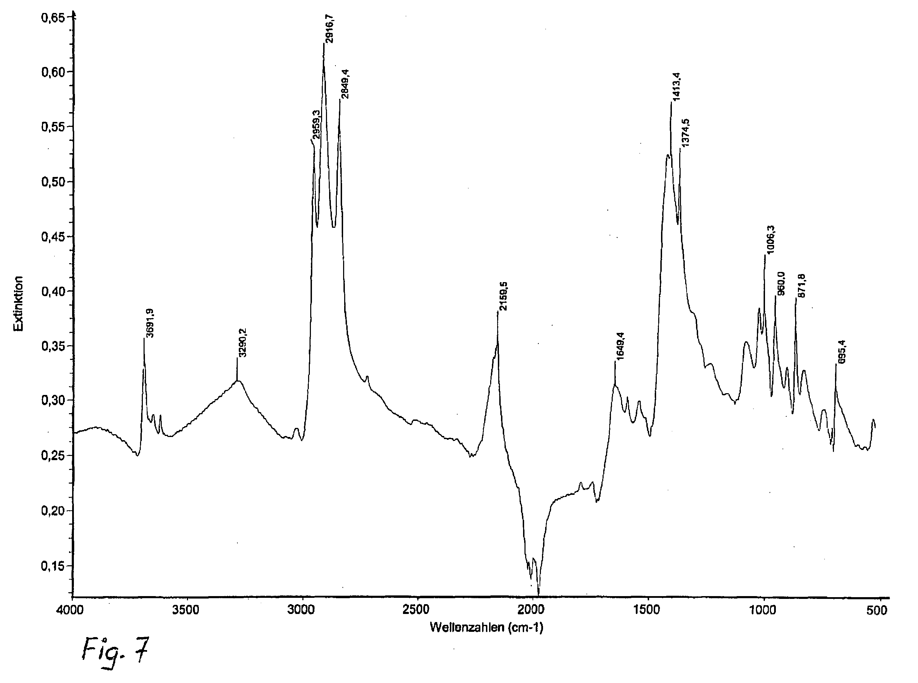

Dagegen zeigt das IR-Spektrum des erfindungsgemäßen Gummis (Mischung 3) gemäß Fig. 7 klar strukturierte bzw. ausgeprägten IR-Spektrallinien. Die spektralen Banden von großer Intensität im Bereich von 1380 cm-1, 1413 cm-1 und 1460 cm-1 sind von Schwingungen der CH3, C-S, CH2 Gruppen verursacht. Dies zeigt das Anwachsen der strukturellen Ordnung und damit eine wachsende stete Wiederholung der relevanten Struktureinheiten des Polymers. Durch den Ersatz des pulverisierten Schwefels durch das flüssige, und deswegen homogen und schnell verteilbare Vernetzungsmittels in dem Rohrgummi erfolgt die Bildung von homogen, sich wiederholenden Struktureinheiten von Schwefel mit der C=C-Polymergruppe. Die Verschiebung der Bande bei 1724 cm-1 (Mischung 1) zu 1649 cm-1 (Mischung 3) zeigt die Verstärkung der Bindungen R-NH, C=C, C=O dem neuen Gummi.

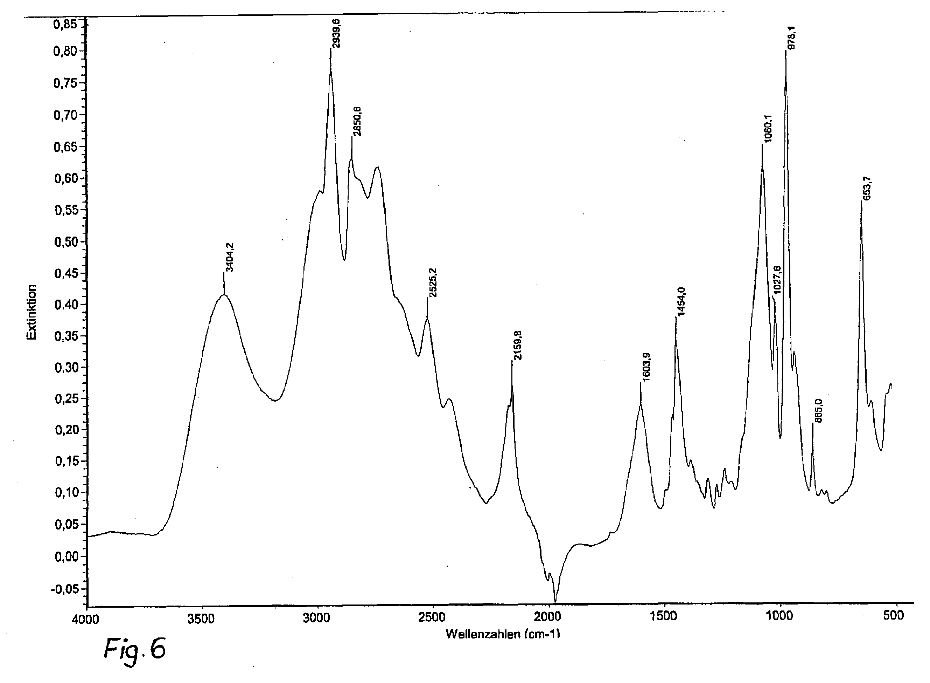

Der erfindungsgemäße Vernetzer aus Piperidin und Schwefel wird durch das IR-Spektrum gemäß Fig. 6 gekennzeichnet. Man erkennt durch den Vergleich von Fig. 6 und 7, dass die spektralen Banden von Mischung 3 und des Vernetzers teilweise miteinander kongruent sind, z.B. in die Bänder 1380 cm-1 - 1460 cm-1, 1600-1650 cm-1, 2850-2960 cm-1.

### Beispiel 3

### Materialtechnische Untersuchungen

**Tabelle 1: Grundrezept der hergestellten Elastomermischungen, die teilweise auch für die spektroskopischen Untersuchungen verwendet wurde.**

| **Bezeichnung** | **Massenanteile (phr)** |
|---|---|
| Kautschuk SMR-L | 100 |
| Zinkoxid | 5 |
| Ruß N 330 | 50 |
| Alterungsschutzmittel IPPD | 1 |
| Stearinsäure | 2 |
| Beschleuniger CBS | 0,25 |

### Mischung 1:

| | |
|---|---|
| Vernetzer: | Schwefel |
| Vernetzer Konzentration: | 1,5 phr |
| Vulkanisationszeit: | 20 Minuten |

### Mischung 2:

| | |
|---|---|
| Vernetzer: | erfindungsgemäßer Vernetzer aus Piperidin und Schwefel |
| Vernetzer Konzentration: | 0,75 phr |
| Vulkanisationszeit: | 25 Minuten |

### Mischung 3:

| | |
|---|---|
| Vernetzer: | erfindungsgemäßer Vernetzer aus Piperidin und Schwefel |
| Vernetzer Konzentration: | 1,5 phr |
| Vulkanisationszeit: | 25 Minuten |

### Mischung 4:

| | |
|---|---|
| Vernetzer: | erfindungsgemäßer Vernetzer aus Piperidin und Schwefel |
| Vernetzer Konzentration: | 3 phr |
| Vulkanisationszeit: | 20 Minuten |

### Vulkameter-Messungen

Zur Messung der Vulkanisation wurden mit einem Vulkameter durchgeführt. Dabei wird eine definierte Menge einer unvulkanisierten Mischung zwischen zwei Metallkammern Druck und Temperatur ausgesetzt. Die Vulkanisation setzt ein, wobei Brücken im Molekül gebildet werden und die Mischung immer stärker vernetzt. Der untere Teil der Kammer wird um einige Winkelgrade hin- und her bewegt, wobei der obere Kammerteil einen Kraftaufnehmer enthält, der die Auslenkung misst und auf einen Schreiber überträgt. Im Ergebnis erhält man eine Hüllkurve, die den Grad der Vernetzung nach einer bestimmten Zeit wie auch die benötigte Zeit, um das Material zu einem gewünschten Anteil zu vernetzen, wiedergibt.

Die Vulkanisation wurde bei 150 °C in einer Etagenpresse durchgeführt, wobei Platten mit den Abmessungen 2 mm x 186 mm x 186 mm hergestellt wurden.

Die durchgeführten Vulkametermessungen haben die in Tabelle 2 angegebenen Werte ergeben.

Es gilt:
Mₕ - maximales Drehmoment
Eₛ - Scorch-Zeit (Zeit bis zum Einsetzen der Vulkanisation)
T₉₀ - Zeit zum Erreichen von 90 % des Drehmomentanstiegs zwischen niedrigsten Drehmoment und Mₕ

**Tabelle 2: Ergebnisse der Vulkametermessungen**

| | t_{S1} (min:sec) | t_{S2} (min:sec) | t₉₀ (min:sec) | Mₕ (dNm) |
|---|---|---|---|---|
| Mischung 1 | 03:34 | 04:30 | 11:32 | 14 |
| Mischung 2 | 04:02 | 11:16 | 12:31 | 4 |
| Mischung 3 | 01:10 | 01:19 | 03:55 | 15 |
| Mischung 4 | 00:25 | 00:30 | 05:05 | 21 |

Die Messwerte zeigen, dass mit dem erfindungsgemäßen Vernetzer eine deutlich schnellere Vulkanisation mit sehr stabilen Plateau der Kurve als mit dem üblicherweise verwendeten Schwefel erreicht wird.

### Härte Shore A nach DIN 53505:

| | |
|---|---|
| Probekörper: | Scheiben, Durchmesser 36 mm |
| Anzahl der Probekörper | 3 Scheiben, 3 fach geschichtet |

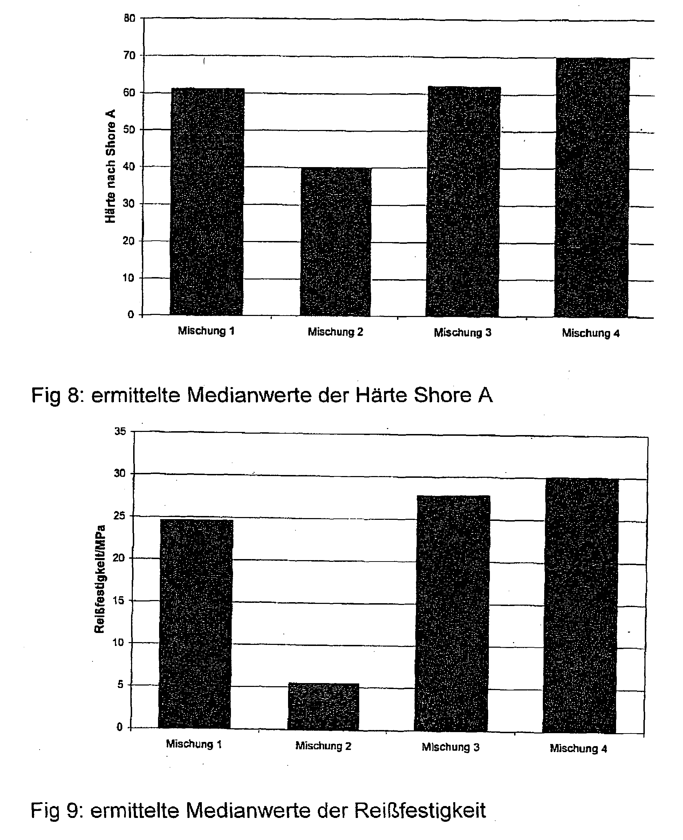

Die Medianwerte der gemessenen Härte sind in Fig. 8 bzw. in Tabelle 3 dargestellt und zusammengefasst.

**Tabelle 3: Ergebnis der Härtemessungen**

| | Härte in Shore A | |
|---|---|---|
| Bezeichnung | Median | Spannweite |
| Mischung 1 | 61 | 2 |
| Mischung 2 | 40 | 4 |
| Mischung 3 | 62 | 5 |
| Mischung 4 | 70 | 1 |

### Reißfestigkeit und Reißdehnung nach DIN 53504:

| | |
|---|---|
| Probekörper: | Schulterstab S2 |
| Anzahl der Probekörper: | je 7 Schulterstäbe |

Die Medianwerte der gemessenen Reißfestigkeit sind in Fig. 9 bzw. in Tabelle 4 dargestellt und zusammengefasst.

**Tabelle 4: Ergebnis der Reißfestigkeit beim Zugversuch**

| | Reißfestigkeit in Mpa | |
|---|---|---|
| Bezeichnung | Median | Spannweite |
| Mischung 1 | 24,6 | 4,4 |
| Mischung 2 | 5,4 | 2,9 |
| Mischung 3 | 27,8 | 4,7 |
| Mischung 4 | 30,1 | 1,2 |

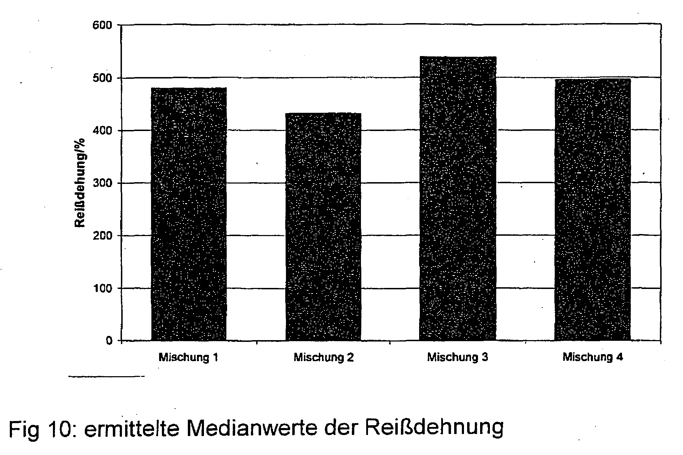

Die Medianwerte der gemessenen Reißdehnung sind in Fig. 10 bzw. Tabelle 5 dargestellt und zusammengefasst.

**Tabelle 5: Ergebnis der Reißdehnung beim Zugversuch**

| | Reißdehnung in % | |
|---|---|---|
| Bezeichnung | Median | Spannweite |
| Mischung 1 | 480 | 63 |
| Mischung 2 | 432 | 136 |
| Mischung 3 | 538 | 91 |
| Mischung 4 | 496 | 42 |

Die Untersuchungen der Härte der Reißfestigkeit sowie der Reißdehnung zeigen bei den vergleichbaren Mischungen 1 und 3 eine deutliche bis sehr deutliche Verbesserung in den Materialeigenschaften der erfindungsgemäßen Gummierzeugnisse.

## Patentansprüche

1. Verfahren zur Herstellung eines Vernetzers zur Vulkanisation, **dadurch gekennzeichnet, dass** eine stickstoffhaltige, heterozyklische Verbindung mit Schwefel umgesetzt wird, wobei die heterozyklische Verbindung ausgewählt ist aus der Gruppe "Piperidin, Pyridin, Anabassin und α,α', ß,'ß, γ,γ'-Dipyridile", und
die Umsetzung bei einer Temperatur unterhalb von 100°C erfolgt.

2. Verfahren nach Anspruch 1, **dadurch gekennzeichnet, dass**
der Schwefel in Pulverform eingesetzt wird.

3. Verfahren nach Anspruch 1 oder 2, **dadurch gekennzeichnet, dass**
während des Mischvorgangs die Temperatur der Mischung unterhalb der Siedetemperatur der heterozyklischen Verbindung gehalten wird.

4. Verfahren nach einem der Ansprüche 1 bis 3, **dadurch gekennzeichnet, dass**
das Verfahren als Batch-Verfahren oder als kontinuierliches Verfahren ausgestaltet ist, wobei entweder der Schwefel vorgelegt und die heterozyklische Verbindung zugesetzt wird, wobei die Zugabe der heterozyklischen Verbindung vorzugsweise tropfenweise, in Schritten oder sonstiger Weise kontinuierlich erfolgt oder die heterozyklische Verbindung vorgelegt und der Schwefel zugesetzt wird, wobei die Zugabe des Schwefels vorzugsweise in Schritten oder sonstiger Weise kontinuierlich erfolgt.

5. Verfahren nach einem der Ansprüche 1 bis 4, **dadurch gekennzeichnet, dass**
unter vermindertem oder erhöhtem Druck gearbeitet wird.

6. Verfahren nach einem der Ansprüche 1 bis 5, **dadurch gekennzeichnet, dass**
unter Kühlung, wobei vorzugsweise die Temperatur unter atmosphärischem Druck nach vollständiger Vermischung der beiden Komponenten bei 65° bis 70° C über einen Zeitraum bis 3 Stunden gehalten wird, gearbeitet wird.

7. Verfahren nach einem der Ansprüche 1 bis 6, **dadurch gekennzeichnet, dass**
zur Mischung von Schwefel und heterozyklischer Verbindung ein bekannter Mischer wie Zentrifugalmischer, Propellermischer, Magnetmischer oder Ultraschallmischer, Vibrationsmischer, Dispergatoren, Turbulenzmischer oder Kombinationen vorgenannter Mischer verwendet wird.

8. Vernetzer für die Vulkanisation, **dadurch gekennzeichnet, dass**
der Vernetzer nach einem Verfahren nach einem der Ansprüche 1 bis 7 hergestellt ist.

9. Vernetzer nach Anspruch 8, **dadurch gekennzeichnet, dass**
dieser ein Vulkanisierungsbeschleuniger ist.

10. Verwendung eines Vernetzers hergestellt nach einem der Ansprüche 1 bis 7 zur Herstellung von Gummierzeugnissen durch Vulkanisation, wobei das Gummierzeugnis ohne weiteren Zusatz von Schwefelpulver vulkanisiert wird.

11. Elastomermischung, **dadurch gekennzeichnet, dass**
die Elastomermischung natürlichen Kautschuk und/oder synthetische Mischungen, vorzugsweise Chloroprenkautschuke, Nitrilkautschuke, Na-Butadienkautschuke und/oder, Styrolkautschuke, sowie einen Vernetzer nach Anspruch 8 oder 9 enthält.

12. Gummierzeugnis hergestellt durch Vulkanisation einer Elastomermischung nach Anspruch 11.

13. Gummierzeugnis hergestellt unter Verwendung eines Vernetzers nach Anspruch 8 oder 9 in Verbindung mit anderen handelsüblichen Vernetzern und/oder Beschleunigern, **dadurch gekennzeichnet, dass** das Gummierzeugnis ohne weiteren Zusatz von Schwefelpulver vulkanisierbar ist.

14. Vulkanisationsprozess zur Herstellung eines Gummierzeugnisses, **dadurch gekennzeichnet, dass**
eine Gummimischung unter Verwendung eines Vernetzers nach Anspruch 8 oder 9 ohne weiteren Zusatz von pulverförmigem Schwefel vulkanisiert wird.

## Claims

1. A process for the preparation of a crosslinker for vulcanization, **characterized in that** a nitrogen-containing heterocyclic compound is made to react with sulfur, wherein the heterocyclic compound is selected from the group of "piperidine, pyridine, anabasine and α,α',β,β',γ,γ'-dipyridile", and
the reaction takes place at a temperature below 100 °C.

2. A process according to claim 1, **characterized in that**
the sulfur is used in powder form.

3. A process according to claim 1 or 2, **characterized in that**
during mixing, the temperature of the mixture is kept below the boiling temperature of the heterocyclic compound.

4. A process according to any one of claims 1 to 3, **characterized in that**
the process is designed as a batch process or as a continuous process, wherein either the sulfur is introduced first and the heterocyclic compound then added, wherein the addition of the heterocyclic compound preferably occurs drop by drop, stepwise or in another continuous manner, or the heterocyclic compound is introduced first and the sulfur then added, wherein the addition of the sulfur preferably occurs stepwise or in another continuous manner.

5. A process according to any one of claims 1 to 4, **characterized in that**
the process is carried out at a reduced or increased pressure.

6. A process according to any one of claims 1 to 5, **characterized in that**
the process is carried out under cooling, wherein preferably the temperature at atmospheric pressure is maintained at 65° to 70 °C over a period of up to 3 hours after the two components have mixed completely.

7. A process according to any one of claims 1 to 6, **characterized in that**
a known mixer such as a centrifugal mixer, propeller mixer, magnetic mixer or ultrasonic mixer, vibratory mixer, dispersers, turbulence mixer or combinations of the above mixers is used to mix the sulfur and the heterocyclic compound.

8. A crosslinker for vulcanization, **characterized in that**
the crosslinker is prepared in accordance with a process according to any one of claims 1 to 7.

9. A crosslinker according to claim 8, **characterized in that**
said crosslinker is a vulcanization accelerator.

10. Use of a crosslinker prepared according to any one of claims 1 to 7 for manufacturing rubber products by vulcanization, wherein the rubber product is vulcanized without any further addition of sulfur powder.

11. An elastomeric mixture, **characterized in that**
the elastomeric mixture contains natural rubber and/or synthetic mixtures, preferably chloroprene rubbers, nitrile rubbers, Na-butadiene rubbers and/or styrene rubbers, as well as a crosslinker according to claim 8 or 9.

12. A rubber product manufactured by vulcanization of an elastomeric mixture according to claim 11.

13. A rubber product manufactured using a crosslinker according to claim 8 or 9 in combination with other commercially available crosslinkers and/or accelerators, **characterized in that** the rubber product can be vulcanized without any further addition of sulfur powder.

14. A vulcanization process for manufacturing a rubber product, **characterized in that**
a rubber mixture is vulcanized using a crosslinker according to claim 8 or 9 without any further addition of powdered sulfur.

## Revendications

1. Procédé de fabrication d'un agent de réticulation pour la vulcanisation, **caractérisé en ce qu'**un composé hétérocyclique contenant de l'azote est mis en réaction avec du soufre, le composé hétérocyclique étant sélectionné dans le groupe « pipéridine, pyridine, anabassine et α, α', β, β', γ, γ'-dipyridile »,
et
la réaction ayant lieu à une température inférieure à 100° C.

2. Procédé selon la revendication 1, **caractérisé en ce que**
le soufre est utilisé sous forme de poudre.

3. Procédé selon la revendication 1 ou 2, **caractérisé en ce que**,
pendant le processus de mélangeage, la température du mélange est maintenue au-dessous de la température d'ébullition du composé hétérocyclique.

4. Procédé selon une des revendications 1 à 3, **caractérisé en ce que** le procédé est constitué en tant que procédé batch ou en tant que procédé continu, soit le soufre étant introduit préalablement et le composé hétérocyclique étant ajouté, l'addition du composé hétérocyclique s'effectuant de préférence goutte à goutte, par étapes ou d'une autre manière en continu, soit le composé hétérocyclique étant introduit préalablement et le soufre étant ajouté, l'addition du souffre s'effectuant de préférence par étapes ou d'une autre manière en continu.

5. Procédé selon une des revendications 1 à 4, **caractérisé en ce que** le travail est effectué sous une pression réduite ou augmentée.

6. Procédé selon une des revendications 1 à 5, **caractérisé en ce que** le travail est effectué en présence d'un refroidissement, la température étant, sous pression atmosphérique, après un mélangeage complet des deux composants, de préférence maintenue à 65° C à 70° C pendant une période allant jusqu'à 3 heures.

7. Procédé selon une des revendications 1 à 6, **caractérisé en ce que**,
pour le mélangeage du souffre et du composé hétérocyclique, il est utilisé un mélangeur connu tel qu'un mélangeur centrifuge, un mélangeur à hélice, un mélangeur magnétique ou un mélangeur à ultrasons, un mélangeur à vibration, des agents dispersants, un mélangeur à turbulence ou des combinaisons de mélangeurs précités.

8. Agent de réticulation pour la vulcanisation, **caractérisé en ce que** l'agent de réticulation est fabriqué selon un procédé selon une des revendications 1 à 7.

9. Agent de réticulation selon la revendication 8, **caractérisé en ce que** celui-ci est un accélérateur de vulcanisation.

10. Utilisation d'un agent de réticulation fabriqué selon une des revendications 1 à 7 pour la fabrication de produits en caoutchouc par vulcanisation, le produit en caoutchouc étant vulcanisé sans ajout supplémentaire de poudre de soufre.

11. Mélange d'élastomère, **caractérisé en ce que**
le mélange d'élastomère contient du caoutchouc naturel et/ou des mélanges synthétiques, de préférence des caoutchoucs de chloroprène, des caoutchoucs de nitrile, des caoutchoucs buna et/ou des caoutchoucs de styrène, ainsi qu'un agent de réticulation selon la revendication 8 ou 9.

12. Produit en caoutchouc fabriqué par vulcanisation d'un mélange d'élastomère selon la revendication 11.

13. Produit en caoutchouc fabriqué en utilisant un agent de réticulation selon la revendication 8 ou 9 en association avec d'autres agents de réticulation et ou accélérateurs du commerce, **caractérisé en ce que** le produit en caoutchouc peut être vulcanisé sans ajout supplémentaire de poudre de soufre.

14. Méthode de vulcanisation pour la fabrication d'un produit en caoutchouc, **caractérisée en ce qu'**un
mélange de caoutchouc est vulcanisé en utilisant un agent de réticulation selon la revendication 8 ou 9 sans ajout supplémentaire de soufre sous forme pulvérulente.
